# EUROPEAN PATENT APPLICATION

(11) **EP 3 932 446 A1**
(43) Date of publication of application: **05.01.2022**
(21) Application number: 21182598.9
(22) Date of filing: 29.06.2021
(51) Int. Cl.: A61M 1/16, A61M 1/32, A61M 1/36, A61B 5/1455, G01N 33/49

(54) **BLOOD LOOP SYSTEM WITH BLOOD OXYGENATION CONTROL**

(30) Priority: 29.06.2020 US 202063045470 P; 24.05.2021 US 202117328335
(71) Applicant: Covidien LP, Mansfield, MA 02048 (US)
(72) Inventor: ESCHBACH, Matthew, Cheshire, CT Connecticut 06410 (US); DIAZ-CHIOSA, Olesea, Naugatuck, CT Connecticut 06770 (US)
(74) Representative: Maschio, Antonio

(57) **Abstract**

A blood loop system for controlling blood oxygen saturation includes a conduit loop (204), a pump (202), a flow cell (206, 208), a matter source (212), an aeration chamber (210), a collection chamber (216) and an oxygen probe (218). The pump (202) is coupled to the conduit loop and positioned to circulate blood through the conduit loop. The flow cell (206, 208) is positioned to measure a characteristic of the blood circulated through the conduit loop. The matter source (212) includes a gas. The aeration chamber (210) is coupled to the conduit loop and is in fluid communication with the matter source (212) to enable the gas to combine with the blood. The collection chamber (216) is in fluid communication with the aeration chamber (210) and is positioned to receive the blood. The oxygen probe (218) is positioned to measure an amount of oxygen in the blood.

## Description

### CROSS-REFERENCE TO RELATED APPLICATIONS

This application claims the benefit of U.S. Provisional Patent Application Serial No. 63/045,470, filed June 29, 2020, the entire contents of which are hereby incorporated by reference herein.

### FIELD

The present technology is generally related to the field of quantification of perfusion and oxygenation levels of bodily tissue.

### BACKGROUND

In surgical settings there is a need to quantify saturation of tissue oxygen to determine an optimally perfused site for transection and anastomosis. Surgical anastomosis can be effectuated using a stapler or suture to prevent fluid leakage. High rates of anastomotic leaks are associated with longer hospital stays and lower quality of life for patients. The cause of leaks can be multifactorial, but frequently include the lack of perfusion. Perfusion carries oxygen, a vital nutrient for wound healing, to tissue throughout the body. A well perfused tissue - and therefore a well oxygenated tissue - is associated with better healing outcomes.

### SUMMARY

Tissue oxygenation is often quantified by two main metrics: peripheral capillary oxygen saturation (SpO2) and tissue capillary bed oxygen saturation (StO2) (e.g., tissue muscle oxygen saturation). SpO2 is measured using near-infrared spectroscopy or pulse oximetry. StO2 is similar to SpO2 pulse oximetry in that it relies on the differential absorption properties of oxygenated and deoxygenated hemoglobin (Hb) to determine the ratio of oxygenated Hb to total Hb in the blood, which is reported as the percentage saturation. Unlike pulse oximetry, which measures oxygen saturation in arterial blood, StO2 measures oxygen saturation in the tissue capillary bed and approximately 80% of the signal is derived from the venous side of circulation. StO2 is a better metric for assessing capillary flow in tissue since it lacks pulsatile flow. By using the optical properties of hemoglobin, (Hb), which change absorption depending on oxygenation state, a light sensor can predict the percentage of oxygenated Hb in the capillaries, giving an indication of perfusion to the anastomosis. This could be accomplished with a spectrometer, but the associated expense can be prohibitive.

Accordingly, there is a need for a user-intuitive, low cost laparoscopic device to objectively quantify perfusion to detect optimal resection sites, and to assess perfusion post operatively. Quantification by a tissue oxygen saturation optical sensor, or a perfusion sensor, may help determine preferable resection sites. In order to assess blood perfusion, a device to measure tissue oxygen saturation (StO2) can be utilized to rule out low perfusion sites. While an in-vivo model can be used, an in-vivo model lacks the ability to control blood oxygen saturation, introduces excessive data due to noise, limits the number of samples that may be collected, and has significant scattering.

The techniques of this disclosure generally relate to maintaining and measuring a property of blood such as oxygenation levels using an ex-vivo blood loop system.

According to one aspect, this disclosure provides a blood loop system for controlling blood oxygen saturation. The blood loop system includes a conduit loop, a pump, a flow cell, a matter source, an aeration chamber, a collection chamber and an oxygen probe. The pump is coupled to the conduit loop and positioned to circulate blood through the conduit loop. The flow cell is positioned to measure a characteristic of the blood circulated through the conduit loop. The matter source includes a gas. The aeration chamber is coupled to the conduit loop and is in fluid communication with the matter source to enable the gas to combine with the blood. The collection chamber is in fluid communication with the aeration chamber and is positioned to receive the blood. The oxygen probe is positioned to measure an amount of oxygen in the blood.

In aspects, the flow cell may be configured to take optical measurements of the aerated blood.

In aspects, the blood loop system may include a thermometer configured to measure a temperature of the blood in the collection chamber and a heater for heating the blood in the collection chamber.

In yet another aspect, the blood loop system may include a computing device in electrical communication with the matter source, the oxygen probe, the flow cell, or combinations thereof. The computing device may be configured to control an amount of blood oxygen saturation in the blood based on signals received from one or both of the flow cell or the oxygen probe.

In other aspects, the pump may be a peristaltic pump configured to circulate the blood continuously through the flow cell.

In aspects, the blood loop system may further include a mixer configured to stir the blood.

In further aspects, the mixer may be configured to stir the blood from about zero revolutions per minute to about sixty revolutions per minute.

In aspects, the mixer may include a magnetic stirrer.

In another aspect, the aeration chamber may be positioned at a higher elevation than the collection chamber

In yet other aspects, the gas may include oxygen and/or nitrogen that combine with the blood to aerate the blood.

According to one aspect, this disclosure provides a method for controlling blood oxygen saturation. The method includes pumping blood through a conduit loop, measuring a characteristic of the blood with a flow cell connected to the conduit loop, supplying oxygen and nitrogen to the blood within an aeration chamber coupled to the conduit loop, receiving the blood in a collection chamber, and measuring an amount of oxygen in the blood with an oxygen probe.

In further aspects, the method may include optically measuring an oxygen saturation of the blood with the flow cell.

In aspects, the method may include measuring a temperature of the blood with a thermometer.

In another aspect, the method may include maintaining a predetermined temperature of the blood with a heater positioned to heat the blood within the collection chamber.

In further aspects, the method may include electrically communicating with the oxygen probe and the flow cell with a computing device.

In other aspects, the method may include continuously circulating blood through the conduit loop with a peristaltic pump.

In aspects, the method may include stirring the blood with a mixer disposed in the collection chamber. The method may include stirring the blood with the mixer at a rate of about zero revolutions per minute to about sixty revolutions per minute.

In further aspects, the method may include maintaining the aeration chamber at a higher elevation than the collection chamber.

In one aspect, the method may include heating the blood with a heater positioned adjacent to the collection chamber.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Objects and features of this disclosure will become apparent to those of ordinary skill in the art when descriptions of various aspects thereof are read with reference to the accompanying drawings, of which:
FIG. 1 is a diagram of an oxygenated and deoxygenated hemoglobin;
FIG. 2 is a perspective view of a blood loop system with blood oxygenation control, according to aspects of this disclosure;
FIGS. 3A and 3B are perspective views of another blood loop system, according to aspects of this disclosure;
FIG. 3C is a sectional view of an oxygenator shown in the blood loop system of FIGS. 3A and 3B, according to aspects of this disclosure;
FIG. 4 is a flow diagram of a method for maintaining and measuring a characteristic of a blood sample, according to aspects of this disclosure;
FIG. 5 is a graph of a moral extinction coefficient of oxygenated and deoxygenated hemoglobin, according to aspects of this disclosure;
FIG. 6 is a graph of an absorbance response for varying levels of oxygenated blood, according to aspects of this disclosure;
FIG. 7 is a graph of an absorbance response of deoxygenated blood, according to aspects of this disclosure; and
FIG. 8 is a graph of a gaussian regression algorithm comparing predicted and actual StO2 levels of a sample of blood, according to aspects of this disclosure.

Further details and aspects of various aspects of this disclosure are described in more detail below with reference to the appended figures.

### DETAILED DESCRIPTION

This disclosure relates to a blood loop device system with the ability to control blood oxygen saturation in an ex-vivo model.

Aspects of this disclosure are described in detail with reference to the drawings, in which like reference numerals designate identical or corresponding elements in each of the several views.

Although this disclosure will be described in terms of specific aspects, it will be readily apparent to those skilled in this art that various modifications, rearrangements, and substitutions may be made without departing from the spirit of the disclosure.

For purposes of promoting an understanding of the principles of this disclosure, reference will now be made to exemplary aspects illustrated in the drawings, and specific language will be used to describe the same. It will nevertheless be understood that no limitation of the scope of this disclosure is thereby intended. Any alterations and further modifications of the inventive features illustrated herein, and any additional applications of the principles of this disclosure as illustrated herein, which would occur to one skilled in the relevant art and having possession of this disclosure, are to be considered within the scope of this disclosure.

This disclosure relates to a blood loop system with the ability to control blood oxygen saturation and methods thereof. This disclosure enables controlled and precise measurements, reduced noise, large sample size collections, repeatable results, and simulations of oxyhemoglobin and deoxyhemoglobin conditions.

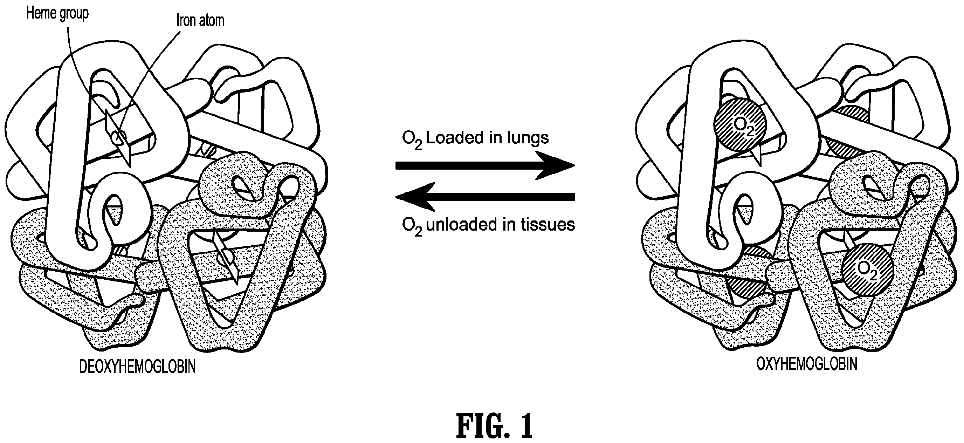

Referring to FIG. 1, blood cell hemoglobin, with and without oxygen, are shown. Hemoglobin (Hb) is an oxygen transport metalloprotein in red blood cells that is composed of four polypeptide chains of two types, each of which has iron containing structures known as a heme group. The iron is the site to which a molecule of oxygen binds (FIG. 1). The complex of hemoglobin and bound oxygen is termed oxyhemoglobin; a hemoglobin without bound oxygen is deoxyhemoglobin. A single hemoglobin may carry four (4) oxygen molecules. When blood carrying the oxyhemoglobin reaches tissues, the oxygen molecules dissociate from the hemoglobin and diffuse to the cells. When all oxygen binding sites on a hemoglobin are occupied, the hemoglobin molecule is said to be 100% saturated. Changes in the hemoglobin structure from oxyhemoglobin to deoxyhemoglobin may be optically detected. The amount of oxyhemoglobin in a blood sample, or the level of oxygen of a blood sample, may be measured by determining the amount of light absorbed by the blood sample using a spectrometer, similar to that disclosed in U.S. Patent Application Publication No. 2006/0135861, the entire contents of which are incorporated by reference herein. Varying wavelengths may be associated with varying oxygen saturation levels by measuring the absorbance response of the blood. A blood loop system and methods for maintaining and measuring characteristics of blood, in accordance with this disclosure, are used to control the oxygen saturation or the level of oxygen infused into the hemoglobin. Using the blood loop system and methods thereof, users are able to determine optimal wavelengths associated with various oxygen saturation levels for developing an StO2 sensor. Comparisons may be made between oxygen saturation levels taken directly using an oxygen probe and the absorbance response of the blood measured using the blood loop system and methods of this disclosure.

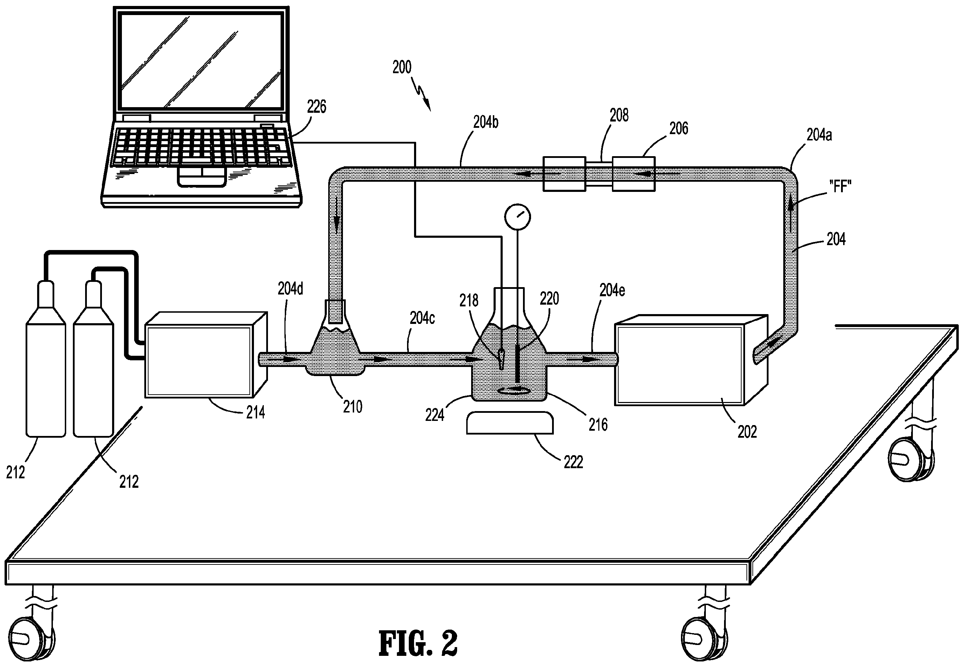

Referring to FIG. 2, a blood loop system 200 for circulating blood and for maintaining and measuring characteristics of the blood is shown. The system operates by enabling blood to flow around a conduit loop formed of one or more conduit loop sections, with individual sections of the conduit loop for testing oxygen levels, oxygenating the blood using gases from controllable matter sources, and measuring the oxygen levels in the aerated blood with an oxygen probe.

The system 200 includes a conduit loop 204 and one or more pumps 202, flow cells 206, aeration chambers 210, matter sources 212, collection chambers 216, and oxygen probes 218. The flow cells 206 may include optical sensors 208. The system 200 may further include one or more gas mixers 214, thermometers 220, heaters 222, mixers 224, and/ or computing devices 226. The conduit loop 204 enables blood to be in fluid communication with the pumps 202, flow cells 206, aeration chambers 210, matter sources 212, collection chambers 216 and oxygen probes 218. The pump 202 may be coupled to the conduit loop 204 and positioned to pump blood through the flow cells 206, aeration chambers 210, collection chambers 216, and/or oxygen probes 218 positioned along the conduit loop. The matter sources 212 and/or gas mixers 214 are in fluid communication with the aeration chambers 210. The thermometers 220 and mixers 224 may be disposed in the collection chambers 216. The heaters 222 may be disposed in or adjacent to the collection chambers 216. The computing devices 226 may be in electrical communication with the pumps 202, flow cells 206, matter sources 212, oxygen probes 218, thermometers 220, heaters 222, and/or mixers 224. The pump 202 circulates blood through conduit loop section 204a of conduit loop 204 to flow cell 206 where a characteristic of the blood is measured. The flow cell 206 includes an optical sensor 208 for measuring the blood using, for example, a spectrometer. The blood then flows from the flow cell 206 through conduit loop section 204b to an aeration chamber 210. The aeration chamber 210 introduces a gas from a matter source 212, such as oxygen and/or nitrogen gases, to the blood sample. The gas from matter source 212 may first pass through a gas mixer 214 to mix multiple gases before introduction to the blood in the aeration chamber 210. The blood exits the aeration chamber 210 through conduit loop section 204c to a collection chamber 216 where an oxygen probe 218 measures oxygen saturation levels of the blood. The oxygen saturation levels measured by the oxygen probe 218 provide a basis for comparing the oxygen saturation levels observed in the absorbance response measured by the flow cell 206. A mixer 224 stirs the blood in the collection chamber 216. A heater 222 is used to maintain the temperature of the blood in the collection chamber 216, and a thermometer 220 is used to monitor the temperature. Computing devices 226 are configured to control the oxygen probes 218, matter sources 212, and/or flow cells 206. Computing devices 226 can be configured to control gas mixers 214, thermometers 220, heaters 222, mixers 224, pumps 202, and/ or flow cells 206. The pump 202 pumps the blood from collection chamber 216 through conduit 204e back to conduit 204a and flow cell 206 thus forming a blood loop system.

With continuing reference to FIG. 2, pump 202 of system 200 circulates blood through one or more conduits 204 in a direction indicated by arrows "FF." Pump 202 may be a peristaltic pump. The pump 202 may be a variable speed pump. Any suitable pump control method or flow rate may be used. For example, the blood may be circulated from about a rate of one (1) milliliters per hour (ml/h) to about one thousand (1000) ml/h. In aspects, the blood may be pumped at a rate of about seventy (70) ml/h to about ninety (90) ml/h. The pump 202 may be controlled by a computer 226 or a dedicated pump control panel. For example, a peristaltic pump with a built-in display and individual controls as known by a person of ordinary skill in the art may be provided.

A flow cell 206, which may be positioned downstream of the pump 202 at the opposite end of conduit loop section 204a, enables the system 200 to measure a characteristic of the blood. The flow cell 206 may include an optical sensor 208, such as a spectrometer, for measuring the oxygen saturation levels of the blood. The blood in the flow cell 206 may be measured using a spectrometer or other suitable methods known by a person of ordinary skill in the art. For more detailed description of such measurement, reference can be made to U.S. Patent Application Publication No. 2006/0135861, incorporated by reference hereinabove. The oxygen levels may be measured using reflectance values of the blood by a reflective optical sensor flow cell. The flow cell 206 may accommodate both transmissive and reflective interrogation modes so that several spectrometer systems may be used in parallel. In aspects, the optical sensor 208 optically measures the light absorbance of the blood so the system 200 (e.g., computing device 226 and/or controller thereof) can evaluate the light (e.g., the entire broad spectrum of light or portions thereof). The optical sensor 208 may be coupled to computing device 226 (e.g., locally via wired or wireless connection) an/or to a separate computing device, server, and/or network (not shown), which may include memory, storage device(s), controllers (e.g., software) for measuring, tracking, and/or analyzing data from the system and/or blood therein. The data may be measured and/or processed in real time (e.g., continuously) and/or at specified intervals. The data may include visible and/or infrared light ranges.

In aspects, the flow cell 206 may utilize principles of absorbance spectroscopy to measure an amount of a dissolved substance in a matter. Specific wavelengths associated with the dissolved substance may be isolated. Isosbestic points may be utilized to ensure that measurements are correctly being recorded. The isosbestic points are overlaps between oxygenated and deoxygenated blood spectra, where total absorbance may not change regardless of its saturation. And, the amount of oxyhemoglobin in a tissue may be determined using identified wavelengths of light and their intensities associated to various oxygenation levels.

As the blood exits flow cell 206, conduit loop section 204b guides the blood to an aeration chamber 210. The aeration chamber 210 may be a long neck flask or a beaker. Any suitable chamber may be used. The end of conduit loop section 204b may be inserted in the aeration chamber 210 such that blood drips from the end of conduit loop section 204b into the aeration chamber 210. Conduit loop section 204b may or may not be in fluid communication with the blood of the aeration chamber 210 as shown in FIG. 2. In aspects, conduit end 204b is inserted such that a gap exists between conduit loop section 204b and the opening of aeration chamber 210 to permit the escape of excess gas. It is contemplated that the conduit loop section 204b fills the opening of the aeration chamber 210 such that there is no gap between the two, and a separate opening enables excess gas to escape. One or more aeration chambers 210 may be positioned downstream of the flow cell 206. The one or more aeration chambers 210 may be connected to one or more matter sources 212 including oxygen and/or nitrogen gases. The one or more matter sources 212 may be used to aerate the blood in the aeration chamber 210. The blood loop system 200 further includes a collection chamber 216 in fluid communication with the aeration chamber 210 so that the aerated blood can flow into the collection chamber 216. The aeration chamber 210 may be positioned at an elevation higher than the elevation of the collection chamber 216 to impart a gravity feed.

A matter source 212 including a gas may be connected to aeration chamber 210 via a conduit 204d. In various aspects, matter source 212 includes oxygen and/ or nitrogen gas. Gas from the matter source 212 is dissolved into the blood in the aeration chamber to form aerated blood. The gas from matter source 212 may be introduced into the blood in the aeration chamber 210 below conduit loop section 204c to allow the gas to dissolve into the blood before the blood travels further downstream along conduit loop section 204c. The matter source 212 includes controls to increase, decrease, and/or maintain the flow of the gas into the aeration chamber 210. The matter source 212 may be controlled by any known methods, such as manually operable valves or digital control systems. Matter source 212 may be connected to and controlled by computing device 226. It is contemplated that matter source 212 is connected to a mixer 214 so that multiple gases from matter sources 212 are mixed to achieve a desired gas mixture. The gas mixture flows from the mixer 214 through conduit loop section 204d to the aeration chamber 210. For example, oxygen and nitrogen gases from matter source(s) 212 may be mixed to achieve a homogenous gas that is 80% oxygen and 20% nitrogen before flowing to the aeration chamber 210. The gases from matter source(s) 212 mix with the blood in the aeration chamber 210 to form an aerated blood. The aerated blood flows from the aeration chamber 210 through conduit loop section 204c to a collection chamber 216.

With continuing reference to FIG. 2, collection chamber 216 is positioned downstream of the aeration chamber 210 for receiving the aerated blood. The collection chamber 216 may be a baffled Erlenmeyer flask, a beaker, and/or any suitable chamber. The collection chamber 216 may be positioned at an elevation lower than the elevation of the aeration chamber 210 such that the blood may be enabled by gravity to flow from the aeration chamber 210 to the collection chamber 216. It is contemplated that one or more second pumps may be provided to enable the flow of blood from the aeration chamber to the collection chamber. In aspects, the collection chamber 216 is closed to the environment.

With continuing reference to FIG. 2, the blood loop system 200 further includes an oxygen sensor 218 to measure an amount of oxygen in the aerated blood. The oxygen sensor 218 may be positioned at any suitable location along the blood loop system 200. In aspects, the oxygen sensor 218 is an oxygen probe 218 and is inserted into the blood of the collection chamber 216. The oxygen probe 218 may be connected to computing device 226 for calibrating the oxygen probe 218 and tracking and/or analyzing oxygen levels of the aerated blood. The oxygen probe 218 may measure oxygen levels for a basis of comparison by computing device 226 with the results of the flow cell oxygen level measurements. For example, the oxygen probe 218 may be calibrated by first measuring the oxygen levels of the blood with no gas from matter source 212 dissolved into the blood, and then measuring a second time at a chosen level with a known amount of gas from matter source 212 introduced to form aerated blood. Comparing, with computing device 226, the oxygen levels measured by the oxygen probe 218 to the oxygen levels determined using the flow cell 206 provides a basis for developing an StO2 sensor and algorithm. The oxygen probe 218 may be positioned away from the aeration chamber 210 so as not to read true oxygen levels of the blood loop system 200, rather the oxygenation levels of the blood itself.

The blood loop system 200 may include a thermometer 220 for monitoring the temperature of the blood and a heater 222 for regulating the temperature of the blood. In aspects, the thermometer 220 is disposed in the collection chamber 216. It is envisioned that at least one thermometer 220 be positioned at any point along the blood loop system 200 as desired for monitoring the temperature of the blood. The heater 222 may maintain the temperature of the blood from about 96 °F (35.5 °C) to about 105 °F (40.5 °C). The heater 222 may be adjacent to the collection chamber 216. For example, the heater 222 may be a hot plate positioned below the collection chamber 216. It is envisioned that at least one suitable device for heating blood can be positioned at any point along the blood loop system 200 as desired for maintaining the temperature of the blood. For example, the conduit 204 may be heated via an electrical tracing heater. In another example, a heater 222 and thermometer 220 may be placed upstream of the flow cell 206 for heating and monitoring the blood circulated by the pump 202. The heater 222 and/or the thermometer 220 may be controlled and/or monitored by a computing device 226.

With continuing reference to FIG. 2, the blood loop system 200 further includes a mixer 224 for stirring the blood. The mixer 224 may be a magnetic mixer or any suitable mixer. The mixer 224 may be set to stir the blood at any suitable mixing speed such as from about zero (0) revolutions per minute (rpm) to about one thousand (1,000) rpm. For example, a magnetic mixer may be set to stir the blood in the collection chamber 216 at a speed from about zero (0) rpm to about sixty (60) rpm. Mixer 224 may be set to any suitable speed such that blood clotting does not impede measurements taken by either the oxygen sensor 218 or flow cell sensor 208. The mixer may be operable with or without the use of a computing device 226.

In various aspects, the blood loop system 200 includes multiple collection chambers 216 positioned along any section of conduit loop 204. The multiple collection chambers 210 may each include one or more oxygen sensors 218, mixers 224, heaters 220, and/or thermometers 222. It is envisioned that multiple aeration chambers 210 be used in conjunction with one or more matter sources 212. Multiple flow cells 204 may be dispersed throughout the blood loop system 200. A single computing device 226 or multiple computing devices 226 may be used to control and/or operate the pump 202, flow cell 206, aeration chamber 210, matter source 212, gas mixer 214, oxygen probe 218, thermometer 220, heater 222, and/or mixer 224. The blood loop system 200 of this disclosure may be used to oxygenate the blood to various saturation levels. The blood loop system 200 enables simulation of various oxygenated and de-oxygenated conditions. For example, a matter source 212 is regulated such that varying the amount of oxygen and/ or nitrogen gases flowing from the matter source 212 to be combined with the blood in the aeration chamber 210 produces blood oxygen saturations of 10%, 75% or 93%. The oxygen saturation level of the blood is measured using the oxygen probe 218 in the collection chamber 216. The oxygen sensor 218 may be disposed in the collection chamber 216 so as not to measure the total oxygen in the blood loop system, whereas an ill-placed oxygen sensor 218 may measure the levels of the oxygen and/or nitrogen gas supplied by the matter sources 212 not absorbed by the blood and the gas absorbed by the blood. The data from the flow cell may be recorded to correspond with the levels of oxygen as measured by the probe. The flow cell optical sensor 208 may provide the level of oxygen in the blood using algorithms associating reflectance to various oxygen saturation levels. For example, the blood oxygen saturation may be regulated to achieve 77% oxygenation of the blood as verified by the oxygen probe, and then the oxygen levels may be measured again by the flow cell sensor via an algorithm associating the reflectance of the blood to the oxygen saturation levels.

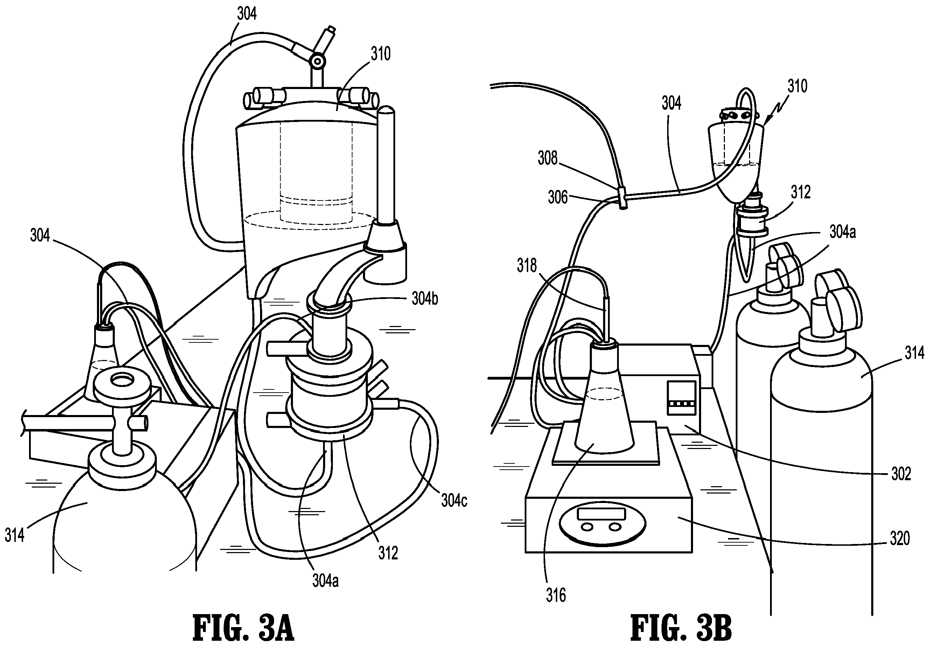

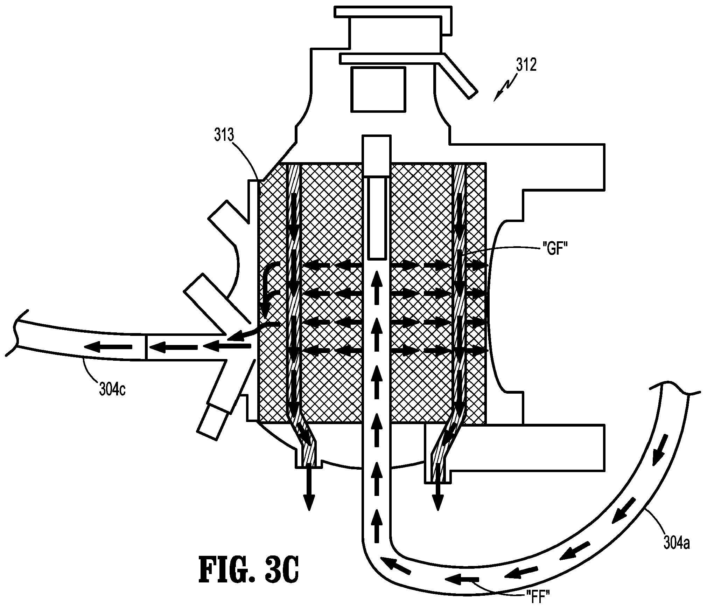

Referring to FIGS. 3A-C, a blood loop system including an aeration chamber 310 with an oxygenator 312 is illustrated. A pump 302 circulates blood through conduit loop 304 to a flow cell 306. The flow cell 306 is configured to measure one or more characteristics of blood, such as blood oxygen saturation. Flow cell 306 may include an optical sensor 308 for measuring blood oxygen saturation. Blood is pumped from flow cell 306 to aeration chamber 310. Aeration chamber 310 may include an oxygenator 312 with an oxygen permeable layer 313 for diffusing oxygen into the blood. The oxygen permeable layer 313 may prevent excess gas from entering and forming bubbles in the blood loop system. A matter source 314 including oxygen and/or nitrogen gases is in fluid communication with the aeration chamber 310 and oxygenator 312. For example, a matter source 314 including nitrogen and/or oxygen gas is connected to the oxygenator 312 by conduit loop section 304b separated by the oxygen permeable layer 313, and the blood from conduit loop 304 at conduit loop section 304a enters the oxygenator at another end. The gas from matter source 314 diffuses through the oxygen permeable layer 313 of the oxygenator 312 to form an aerated blood. The aerated blood flows through conduit loop section 304c from the aeration chamber 310 with oxygenator 312 to a collection chamber 316. The collection chamber 316 may be positioned at an elevation lower than that of the aeration chamber 310 with an oxygenator 312 so as to enable blood flow due to gravity. An oxygen probe 318 is positioned in the collection chamber 316 to measure the oxygen saturation of the aerated blood. A mixer 320 may stir the aerated blood in the collection chamber 316. Blood is pumped from the collection chamber 316 back to the flow cell 306 via pump 302.

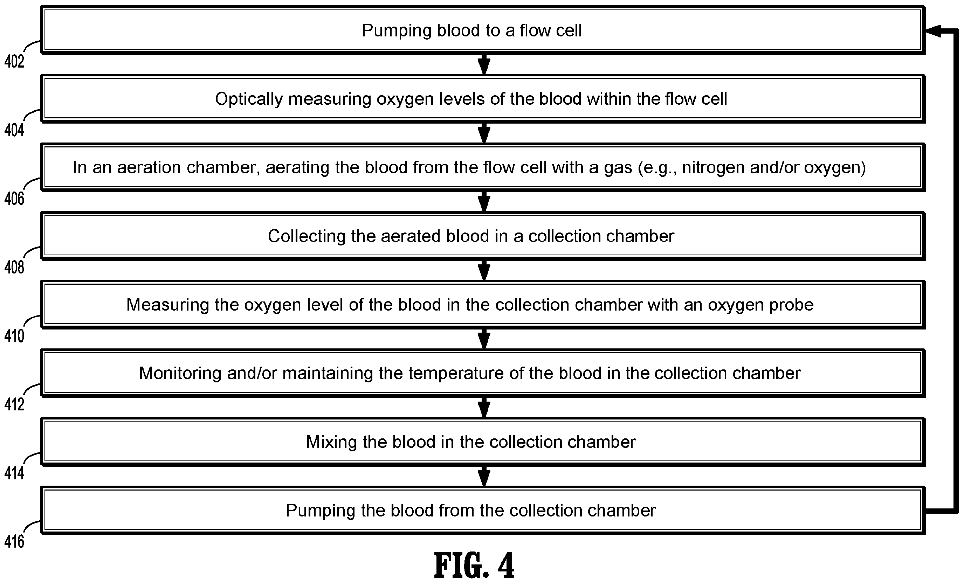

Referring to FIG. 4, a diagram of a method for maintaining and measuring a characteristic of a blood sample according to aspects of this disclosure is shown. By process 402, blood is pumped through a conduit loop 204 to a flow cell 206 (see FIG. 2). Properties of the blood are measured via the flow cell 206 by process 404. For example, at process 404, blood oxygenation levels of the blood in the flow cell 206 may be measured optically via a spectrometer. Blood from the flow cell 206 is pumped to an aeration chamber 210 (FIG. 2), and then aerated and mixed with one or more gases from matter sources 212 (FIG. 2) by process 406 to form an aerated blood. For example, oxygen and/or nitrogen gases from matter sources 212 may be introduced to the blood via an oxygenator 312 (see FIG. 3A-C) in the aeration chamber 210, where an oxygen permeable membrane 313 separates the blood from the gas allowing oxygen to mix into the blood. By process 408, the aerated blood is collected in a collection chamber 216 (see FIG. 2). By process 410, the oxygen levels of the blood in the collection chamber 216 is measured using an oxygen probe 218 (FIG. 2). The temperature of the blood may be maintained and/ or monitored by process 412 using a heater 222, such as a hot plate, and/or a thermometer 220 respectively (see FIG. 2). The blood in the collection chamber 216 may be mixed by process 414. In aspects, the blood is mixed using a magnetic stirrer 224 (FIG. 2) at a speed from about zero (0) rpm to about sixty (60) rpm by process 414. The blood from the collection chamber 216 is then pumped back to the flow cell 206 to repeat the method by process 416, according to aspects of this disclosure. Multiple processes may occur simultaneously. For example, process 412 and process 414 may be concurrently executed such that the blood temperature is monitored by a thermometer 220 and maintained by a heater 222 while stirred by a mixer 224. It is envisioned that each process described herein may occur simultaneously and continuously to allow for constant testing of various blood oxygen saturation levels. Alternatively, and/or additionally, such processes can occur sequentially, randomly, and/or at any suitable interval. The processes may each be executed and/or controlled by a computer program stored on a computing device in electrical communication with the various components and/or devices used in the method. For instance, as described in greater detail below, memory and/or a storing device, which may be supported on computing device or remote server, can include instructions stored thereon, which, when executed by, for example, a processor, cause one or more of the components of the disclosed system to operate (e.g., pump, measure, heat, etc.).

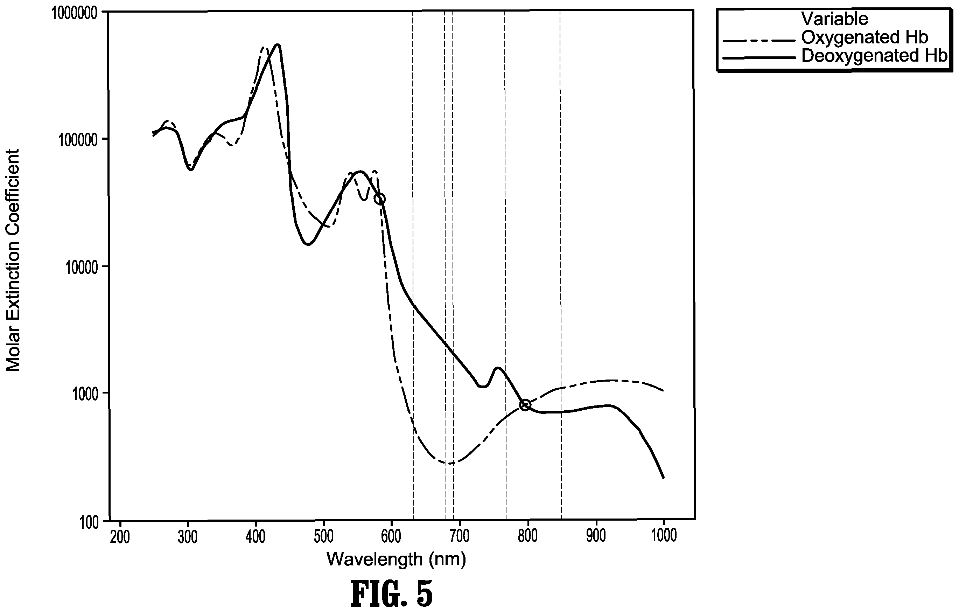

Referring to FIG. 5, a graph of the molar extinction coefficient for oxygenated and deoxygenated hemoglobin (Hb) is shown. Known isosbestic points are seen at wavelengths of 586 nm and at 808 nm. The molecular extinction coefficient for oxygenated Hb for wavelengths from about 580 nm to about 800 nm is smaller than the that for deoxygenated Hb. The disclosed system is configured to determine, for example wavelengths where the disparity between the oxygenated and deoxygenated molar extinction coefficients is greatest. As indicated by FIG. 5, wavelengths between 580 nm and 800 nm, for instance, may be monitored by the disclosed system as a range indicative of a disparity of the molar extinction coefficient between oxygenated and deoxygenated blood.

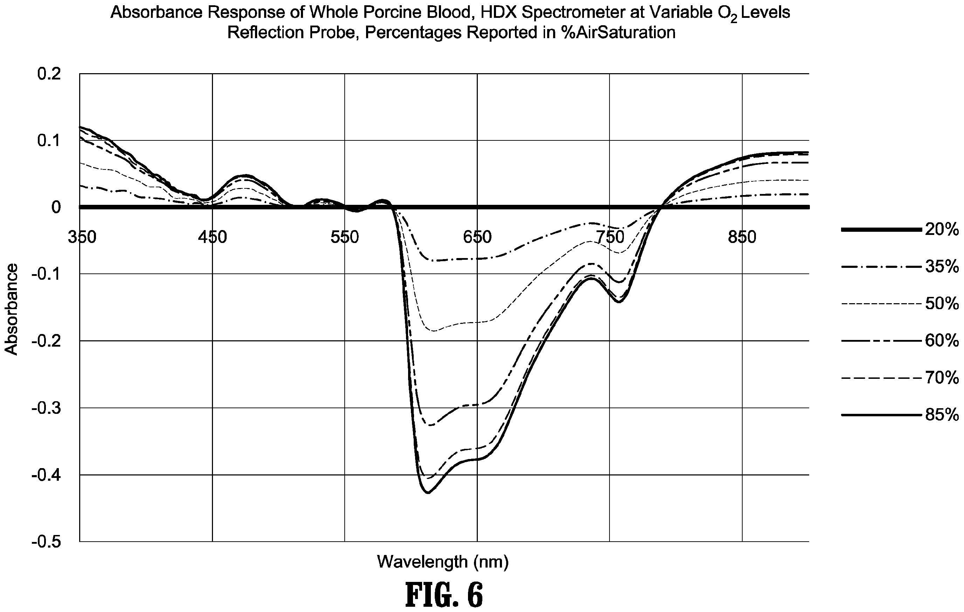

Referring to FIG. 6, an example graph of the absorbance response of whole porcine blood at variable O2 levels using the blood loop system of this disclosure is shown. Porcine blood was circulated through a blood loop system 200 using a pump 202 to pump the blood from a collection chamber 216 to a flow cell 206 (see FIG. 2). The flow 206 cell took optical measurements and provided the absorbance response of the blood. The porcine blood was then pumped to an aeration chamber 210 where it was combined with a gas from a matter source 212 to desired oxygen saturation levels to form aerated porcine blood (see FIG. 2). Thereafter, the aerated porcine blood was fed to the collection chamber 216 via gravity in the blood loop system (see FIG. 2). The temperature of the porcine blood was monitored using a thermometer 220 inserted in the collection chamber 216 and maintained via a heater 222 below the collection chamber 216. The absorbance response is shown for blood oxygenation levels at 20%, 35%, 50%, 60%, 70% and 85%. For all oxygenation levels, there are isosbestic points at 586 nm and 808 nm. Between about 550 nm and about 825 nm wavelengths, absorbance grows stronger with increased oxygenation levels. The disclosed system can be configured to isolate various wavelengths where the differences at each blood oxygenation level are the most pronounced.

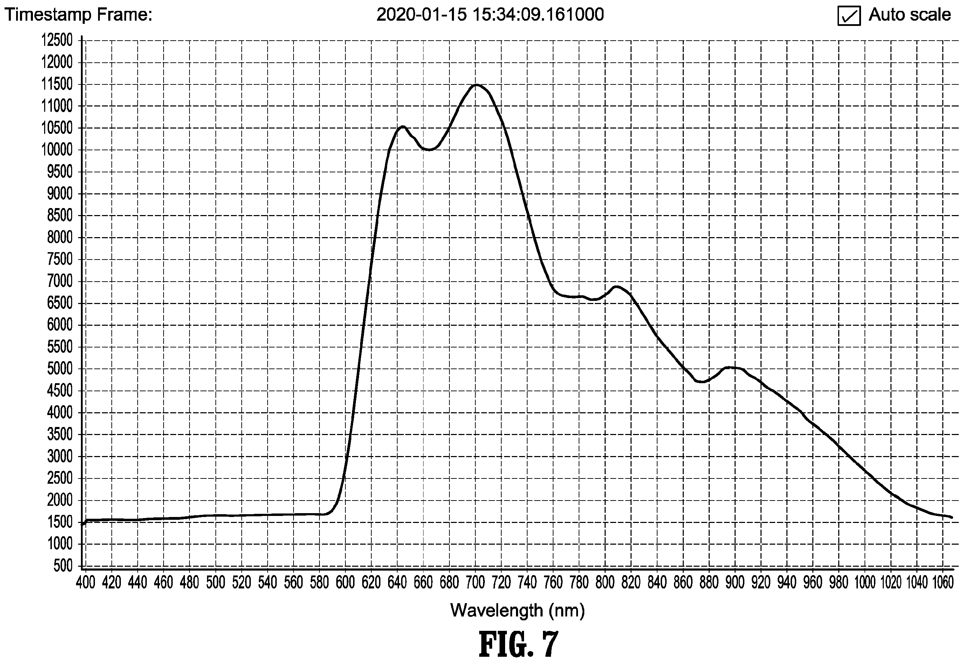

Referring to FIG. 7, a graph of the absorbance response of oxygenated blood is shown. The oxygen saturation level of a blood sample circulated and aerated using the blood loop system of this disclosure was measured. In particular, the disclosed systems are configured to compare the intensity of specific wavelengths of the absorbance response to the intensity of the same wavelength of various other blood samples of varying oxygen saturations to determine StO2 levels of blood in a blood loop system as shown in FIG. 7.

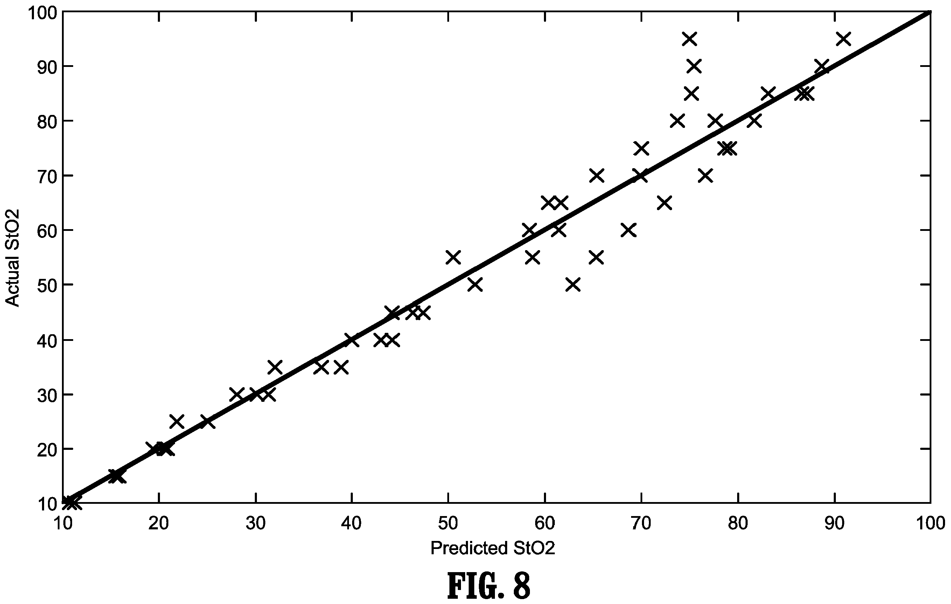

Referring to FIG. 8, an example graph of a gaussian regression algorithm comparing predicted and actual StO2 levels of blood in a blood loop system of this disclosure is shown. Fifty-two (52) different reflection measurements were taken on porcine blood ranging from 10% to 95% oxygen saturation via the blood loop system of this disclosure. Optical reflectance data was obtained using a flow cell configured to take optical measurements using an optical sensor. An algorithm was run using eight (8) input variables, of which the first seven (7) were wavelengths between 575 and 900 nm and the last was an integer between four (4) and seven (7) dictating how many wavelengths of the seven (7) to use for a regression analysis. Three (3) different sets of input parameters were calculated for the regression analysis: first derivatives, second derivatives, and the combination of the two. All were calculated from the reflectance absorbance with respect to wavelength. A suitable regression algorithm may be a gaussian process, but any suitable algorithm may be used. The input parameters used for the gaussian regression shown in FIG. 8 included only the first derivatives. Optical measurements of a blood sample oxygenated to various levels were compared to the actual oxygen saturation levels as measured by the oxygen probe 218 in the collection chamber 216 of the blood loop system 200 (FIG. 2) as shown in FIG. 8. Using the blood loop system of this disclosure allows for control of blood oxygen saturation and a large number of samples to be collected to improve regression analyses and predict StO2 levels.

The phrases "in an aspect," "in aspects," "in various aspects," "in some aspects," or "in other aspects" may each refer to one or more of the same or different aspects in accordance with the present disclosure. A phrase in the form "A or B" means "(A), (B), or (A and B)." A phrase in the form "at least one of A, B, or C" means "(A); (B); (C); (A and B); (A and C); (B and C); or (A, B, and C)."

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

Moreover, the disclosed structure can include any suitable mechanical, electrical, and/or chemical components for operating the disclosed system or components thereof. For instance, such electrical components can include, for example, any suitable electrical and/or electromechanical, and/or electrochemical circuitry, which may include or be coupled to one or more printed circuit boards. As appreciated, the disclosed computing devices and/or server can include, for example, a "controller," "processor," "digital processing device" and like terms, and which are used to indicate a microprocessor or central processing unit (CPU). The CPU is the electronic circuitry within a computer that carries out the instructions of a computer program by performing the basic arithmetic, logical, control and input/output (I/O) operations specified by the instructions, and by way of non-limiting examples, include server computers. In some aspects, the controller includes an operating system configured to perform executable instructions. The operating system is, for example, software, including programs and data, which manages hardware of the disclosed apparatus and provides services for execution of applications for use with the disclosed apparatus. Those of skill in the art will recognize that suitable server operating systems include, by way of non-limiting examples, FreeBSD, OpenBSD, NetBSD^{®}, Linux, Apple^{®} Mac OS X Server^{®}, Oracle^{®} Solaris^{®}, Windows Server^{®}, and Novell^{®} NetWare^{®}. In some aspects, the operating system is provided by cloud computing.

In some aspects, the term "controller" may be used to indicate a device that controls the transfer of data from a computer or computing device to a peripheral or separate device and vice versa, and/or a mechanical and/or electromechanical device (e.g., a lever, knob, etc.) that mechanically operates and/or actuates a peripheral or separate device.

In aspects, the controller includes a storage and/or memory device. The storage and/or memory device is one or more physical apparatus used to store data or programs on a temporary or permanent basis. In some aspects, the controller includes volatile memory and requires power to maintain stored information. In various aspects, the controller includes non-volatile memory and retains stored information when it is not powered. In some aspects, the non-volatile memory includes flash memory. In certain aspects, the non-volatile memory includes dynamic random-access memory (DRAM). In some aspects, the non-volatile memory includes ferroelectric random-access memory (FRAM). In various aspects, the non-volatile memory includes phase-change random access memory (PRAM). In certain aspects, the controller is a storage device including, by way of non-limiting examples, CD-ROMs, DVDs, flash memory devices, magnetic disk drives, magnetic tapes drives, optical disk drives, and cloud-computing-based storage. In various aspects, the storage and/or memory device is a combination of devices such as those disclosed herein.

In some aspects, the controller includes a display to send visual information to a user. In various aspects, the display is a cathode ray tube (CRT). In various aspects, the display is a liquid crystal display (LCD). In certain aspects, the display is a thin film transistor liquid crystal display (TFT-LCD). In aspects, the display is an organic light emitting diode (OLED) display. In certain aspects, on OLED display is a passive-matrix OLED (PMOLED) or active-matrix OLED (AMOLED) display. In aspects, the display is a plasma display. In certain aspects, the display is a video projector. In various aspects, the display is interactive (e.g., having a touch screen or a sensor such as a camera, a 3D sensor, a LiDAR, a radar, etc.) that can detect user interactions/gestures/responses and the like. In some aspects, the display is a combination of devices such as those disclosed herein.

The controller may include or be coupled to a server and/or a network. As used herein, the term "server" includes "computer server," "central server," "main server," and like terms to indicate a computer or device on a network that manages the disclosed apparatus, components thereof, and/or resources thereof. As used herein, the term "network" can include any network technology including, for instance, a cellular data network, a wired network, a fiber optic network, a satellite network, and/or an IEEE 802.11a/b/g/n/ac wireless network, among others.

In various aspects, the controller can be coupled to a mesh network. As used herein, a "mesh network" is a network topology in which each node relays data for the network. All mesh nodes cooperate in the distribution of data in the network. It can be applied to both wired and wireless networks. Wireless mesh networks can be considered a type of "Wireless ad hoc" network. Thus, wireless mesh networks are closely related to Mobile ad hoc networks (MANETs). Although MANETs are not restricted to a specific mesh network topology, Wireless ad hoc networks or MANETs can take any form of network topology. Mesh networks can relay messages using either a flooding technique or a routing technique. With routing, the message is propagated along a path by hopping from node to node until it reaches its destination. To ensure that all its paths are available, the network must allow for continuous connections and must reconfigure itself around broken paths, using self-healing algorithms such as Shortest Path Bridging. Self-healing allows a routing-based network to operate when a node breaks down or when a connection becomes unreliable. As a result, the network is typically quite reliable, as there is often more than one path between a source and a destination in the network. This concept can also apply to wired networks and to software interaction. A mesh network whose nodes are all connected to each other is a fully connected network.

In some aspects, the controller may include one or more modules. As used herein, the term "module" and like terms are used to indicate a self-contained hardware component of the central server, which in turn includes software modules. In software, a module is a part of a program. Programs are composed of one or more independently developed modules that are not combined until the program is linked. A single module can contain one or several routines, or sections of programs that perform a particular task.

As used herein, the controller includes software modules for managing various aspects and functions of the disclosed system or components thereof.

The disclosed structure may also utilize one or more controllers to receive various information and transform the received information to generate an output. The controller may include any type of computing device, computational circuit, or any type of processor or processing circuit capable of executing a series of instructions that are stored in memory. The controller may include multiple processors and/or multicore central processing units (CPUs) and may include any type of processor, such as a microprocessor, digital signal processor, microcontroller, programmable logic device (PLD), field programmable gate array (FPGA), or the like. The controller may also include a memory to store data and/or instructions that, when executed by the one or more processors, cause the one or more processors to perform one or more methods and/or algorithms.

Any of the herein described methods, programs, algorithms or codes may be converted to, or expressed in, a programming language or computer program. The terms "programming language" and "computer program," as used herein, each include any language used to specify instructions to a computer, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

As can be appreciated, securement of any of the components of the disclosed systems can be effectuated using known securement techniques such welding, crimping, gluing, fastening, etc.

Persons skilled in the art will understand that the structures and methods specifically described herein and illustrated in the accompanying figures are non-limiting exemplary aspects, and that the description, disclosure, and figures should be construed merely as exemplary of particular aspects. It is to be understood, therefore, that this disclosure is not limited to the precise aspects described, and that various other changes and modifications may be effectuated by one skilled in the art without departing from the scope or spirit of the disclosure. Additionally, it is envisioned that the elements and features illustrated or described in connection with one exemplary aspect may be combined with the elements and features of another without departing from the scope of this disclosure, and that such modifications and variations are also intended to be included within the scope of this disclosure. Indeed, any combination of any of the disclosed elements and features is within the scope of this disclosure. Accordingly, the subject matter of this disclosure is not to be limited by what has been particularly shown and described.

The invention may be described by reference to the following numbered paragraphs: -
1. A blood loop system for controlling blood oxygen saturation, the blood loop system comprising:
   a conduit loop;
   a pump coupled to the conduit loop and positioned to circulate blood through the conduit loop;
   a flow cell positioned to measure a characteristic of the blood circulated through the conduit loop;
   a matter source including a gas;
   an aeration chamber coupled to the conduit loop and in fluid communication with the matter source to enable the gas to combine with the blood;
   a collection chamber in fluid communication with the aeration chamber and positioned to receive the blood; and
   an oxygen probe positioned to measure an amount of oxygen in the blood.
2. The blood loop system of paragraph 1, wherein the flow cell is configured to take optical measurements of the aerated blood.
3. The blood loop system of paragraph 1, further comprising a thermometer configured to measure a temperature of the blood in the collection chamber and a heater for heating the blood in the collection chamber.
4. The blood loop system of paragraph 1, further comprising a computing device in electrical communication with the matter source, the oxygen probe, the flow cell, or combinations thereof, the computing device configured to control an amount of blood oxygen saturation in the blood based on signals received from at least one of the flow cell or the oxygen probe.
5. The blood loop system of paragraph 1, wherein the pump is a peristaltic pump configured to circulate the blood continuously through the flow cell.
6. The blood loop system of paragraph 1, further comprising a mixer configured to stir the blood.
7. The blood loop system of paragraph 6, wherein the mixer is configured to stir the blood from about zero revolutions per minute to about sixty revolutions per minute.
8. The blood loop system of paragraph 1, wherein the mixer includes a magnetic stirrer.
9. The blood loop system of paragraph 1, wherein the aeration chamber is positioned at a higher elevation than the collection chamber.
10. The blood loop system of paragraph 1, wherein the gas includes oxygen and nitrogen that combine with the blood to aerate the blood.
11. A method for controlling blood oxygen saturation, the method comprising:
   pumping blood through a conduit loop;
   measuring a characteristic of the blood with a flow cell connected to the conduit loop;
   supplying oxygen and nitrogen to the blood within an aeration chamber coupled to the conduit loop;
   receiving the blood in a collection chamber; and
   measuring an amount of oxygen in the blood with an oxygen probe.
12. The method of paragraph 11, further comprising optically measuring an oxygen saturation of the blood with the flow cell.
13. The method of paragraph 11, further comprising measuring a temperature of the blood with a thermometer.
14. The method of paragraph 13, further comprising maintaining a predetermined temperature of the blood with a heater positioned to heat the blood within the collection chamber.
15. The method of paragraph 11, further comprising electrically communicating with the oxygen probe and the flow cell with a computing device.
16. The method of paragraph 11, further comprising continuously circulating blood through the conduit loop with a peristaltic pump.
17. The method of paragraph 11, further comprising stirring the blood with a mixer disposed in the collection chamber.
18. The method of paragraph 17, wherein stirring the blood with a mixer includes stirring the blood at a rate of about zero revolutions per minute to about sixty revolutions per minute.
19. The method of paragraph 11, further comprising maintaining the aeration chamber at a higher elevation than the collection chamber.
20. The method of paragraph 11, further comprising heating the blood with a heater positioned adjacent to the collection chamber.

## Claims

1. A blood loop system for controlling blood oxygen saturation, the blood loop system comprising:
a conduit loop;
a pump coupled to the conduit loop and positioned to circulate blood through the conduit loop;
a flow cell positioned to measure a characteristic of the blood circulated through the conduit loop;
a matter source including a gas;
an aeration chamber coupled to the conduit loop and in fluid communication with the matter source to enable the gas to combine with the blood;
a collection chamber in fluid communication with the aeration chamber and positioned to receive the blood; and
an oxygen probe positioned to measure an amount of oxygen in the blood.

2. The blood loop system of claim 1, wherein the flow cell is configured to take optical measurements of the aerated blood.

3. The blood loop system of claim 1 or claim 2, further comprising a thermometer configured to measure a temperature of the blood in the collection chamber and a heater for heating the blood in the collection chamber.

4. The blood loop system of any preceding claim, further comprising a computing device in electrical communication with the matter source, the oxygen probe, the flow cell, or combinations thereof, the computing device configured to control an amount of blood oxygen saturation in the blood based on signals received from at least one of the flow cell or the oxygen probe.

5. The blood loop system of any preceding claim, wherein the pump is a peristaltic pump configured to circulate the blood continuously through the flow cell.

6. The blood loop system of any preceding claim, further comprising a mixer configured to stir the blood.

7. The blood loop system of claim 6, wherein the mixer is configured to stir the blood from about zero revolutions per minute to about sixty revolutions per minute.

8. The blood loop system of any preceding claim, wherein the mixer includes a magnetic stirrer.

9. The blood loop system of any preceding claim, wherein the aeration chamber is positioned at a higher elevation than the collection chamber.

10. The blood loop system of any preceding claim, wherein the gas includes oxygen and nitrogen that combine with the blood to aerate the blood.

11. The use of a blood loop system according to any preceding claim for controlling blood oxygen saturation, comprising:
the steps of pumping blood through a conduit loop;
measuring a characteristic of the blood with a flow cell connected to the conduit loop;
supplying oxygen and nitrogen to the blood within an aeration chamber coupled to the conduit loop;
receiving the blood in a collection chamber; and
measuring an amount of oxygen in the blood with an oxygen probe.
